# EUROPEAN PATENT SPECIFICATION

(11) **EP 1 374 863 B1**
(45) Date of publication and mention of the grant of the patent: **02.09.2009**
(21) Application number: 02705258.8
(22) Date of filing: 15.03.2002
(51) Int. Cl.: A61K 31/198, A61K 31/401, A61K 31/405, A61K 31/4172, A61P 1/16

(54) **AMINO ACID COMPOSITIONS FOR AMELIORATING LIVER FAILURE**
AMINOSÄUREZUSAMMENSETZUNGEN ZUR LINDERUNG EINER FEHLFUNKTION DER LEBER
COMPOSITIONS D'ACIDES AMINES UTILISEES POUR AMELIORER LE MALFONCTIONNEMENT HEPATIQUE

(30) Priority: 15.03.2001 JP 2001074964
(43) Date of publication of application: 02.01.2004
(73) Proprietor: RIKEN, Wako-shi, Saitama 351-0198 (JP); Meiji Dairies Corporation, Tokyo 136-0075 (JP); Abe, Takashi, Warabi-shi, Saitama 335-0003 (JP)
(72) Inventor: ABE, Takashi, Warabi-shi, Saitama 335-0003 (JP); SAITOU, Masato, Nutrition Science Institute, Higashimurayama-shi, Tokyo 189-0013 (JP)
(74) Representative: Vossius & Partner
(86) International application number: PCT/JP2002/002500
(87) International publication number: WO 2002/074302

(56) References cited:
- EP-A- 0 983 726
- WO-A1-93/21937
- GB-A- 1 507 951
- JP-A- 3 128 318
- JP-A- 6 024 976
- JP-A- 11 302 164
- JP-A- 56 008 312
- JP-A- 63 054 320
- US-A1- 2001 039 264
- ABE T ET AL: "EFFECTS OF VESPA AMINO ACID MIXTURE (VAAM) ISOLATED FROM HORNET LARVAL SALIVA AND MODIFIED VAAM NUTRIENTS ON ENDURANCE EXERCISE IN SWIMMING MICE -IMPROVEMENT IN PERFORMANCE AND CHANGES OF BLOOD LACTATE AND GLUCOSE-" TAIRYOKU KAGAKU, NIHON TAIRYOKU IGAKKAI, TOKYO, JP, vol. 44, no. 2, 1995, pages 225-237, XP001000105 ISSN: 0039-906X
- DATABASE BIOSIS [Online] BIOSCIENCES INFORMATION SERVICE, PHILADELPHIA, PA, US; 1992, ROCCHI E ET AL: "METABOLIC EFFECTS AFTER A TRYPTOPHAN-FREE AMINO ACID MIXTURE IN LIVER CIRRHOSIS CLINICAL IMPLICATIONS" XP002303692 Database accession no. PREV199294083016 & RECENTI PROGRESSI IN MEDICINA, vol. 83, no. 4, 1992, pages 213-217, ISSN: 0034-1193
- PAUMGARTNER: "Therapie innerer Krankheiten" 1999, SPRINGER VERLAG , BERLIN HEIDELBERG NEW YORK , XP002303691 * page 706, paragraph 3 *

## Description

The present invention relates to an amino acid composition or a solution dissolving the amino acid composition therein having a remarkable suppressing effect on the outbreak of functional disorders of the liver when alcohols are excessively taken and more specifically to the amino acid composition or the solution dissolving the amino acid composition therein which is contained in saliva secreted from the larva of a hornet (*Vespa* genus).

The present inventors have devoted themselves to study saliva secreted from the larva of a hornet, thereby identifying formulation of an amino acid composition contained therein, and to investigate applications of the composition.

The investigation revealed that an amino acid composition named as "VAAM" among the various amino acid compositions contained in the saliva exhibited an action for accelerating kinesthetic functions (Japanese patent No.2518692). The action for accelerating the kinesthetic functions includes muscle power durability, increase of nutrition and toughness, nutrition supply and weariness recovery.

A liver which is the largest organ contained in digestive organs has functions of storing glycogen as carbohydrate, generating and secreting bile, detoxifying toxic compounds and synthesizing proteins from amino acids. The burdens to the liver proportionally increase with these important functions.

Intemperance such as drinking, smoking and staying-up-late may apply excessive burdens to the liver, thereby generating liver insufficiencies including cancer and cirrhosis

Conventionally, synthesized medicines were administrated to a patient having the above diseases. Even if the effectiveness of the synthesized medicines is excellent, these medicines are usually toxic and extend ill-effects to other organs. Accordingly, the essential healing cannot be reached by the use of the above synthesized medicines.

EP-A-0 983 726 and US 2001/039264 relates to an amino acid-sugar composition developed on the basis of the knowledge obtained from a study of a composition which comprises amino acids contained in the saliva secreted by the larvae of a wasp (belonging to the genus Vespa) and more particularly to an amino acid-sugar composition as well as a liquid complement containing the same, which show effects of compensating the reduction of the blood level of amino acids associated with severe exercise, of improving the exercise, of reducing the degree of fatigue after exercise and of recovering from fatigue.

The paper of Abe T. et al ("Effects of vespa amino acid mixture (VAAM) isolated from hornet larval saliva and modified VAAM nutrients on endurance exercise in swimming mice - improvement in performance and changes of blood lactate and glucose-"), Nihon Tairyoku Igakkai, Tokyo, JP, vol. 44, no. 2, 1995, pages 225 - 237 discloses VAAM and VAAM analogue mixtures lacking tryptophane.

Rocchi et al ("Metabolic effects after a tryptophan-free amino acid mixture in liver cirrhosis clinical implications") XP002303692 Database BIOSIS accession no. PREV199294083016 refers to the use of an amino acid mixture lacking tryptophan in the treatment of patients suffering from liver cirrhosis.

Paumgartner: "Therapie innerer Krankheiten" 1999, Springer Verlag, Berlin Heidelberg New York, XP002303691 discloses that plasma tryptophane is elevated in patients suffering from liver cirrhosis.

GB-A-1 507 951 A relates to an amino acid formulation suitable for administration to human patients with liver disease.

JP 56 008 312 A pertains to an amino acid pharmaceurical preparation capable of correcting an amino acid pattern of a patient in invasion and effective as a proper nutrient supply source, comprising specific ratios of specified essential amino-acids and unessential amino- acids.

JP 63 054 320 A refers to a food or drug composition having anti-alcoholic disease action, containing alanine and glutamine in specific molar ratio.

WO 93 21937 A1 discuss protein hydolyzates having particularly desired characteristics.

JP-A-6 024 976 relates to a composition for transfusion solution having an immediate effects on rise of free fatty acid in blood, lowering a lactic acid value in a short time, having improving action by intravenous injection on increase inhibition of lipid in blood or liver and on reduction of lactic acid decomposing ability due to liver function disorder caused by alcohol or cancer.

JP 11 302 164 A pertains to a safe amino acid-containing transfusion capable of supplying a sufficient quantity of glutamine and having well-balanced compounding quantities of other amino acids, excellent in supplementing amino acids and having excellent storage stability and solubility.

JP-A-3 128 318 discoloses a composition containing specific amounts of amino acids such as threonine, proline, glycine, valine, isoleucine, leucine and tyrosine and less than a specific amount of methionine, etc., acting on the muscle and nervous system and effective on lowering the concentration of lactic acid in blood.

In view of the foregoing, an object of the present invention is to provide a nature-originated amino acid composition or an amino acid composition solution which has a prominent action for suppressing the outbreak of functional disorders of a liver when alcohols are excessively taken, and is substantially free of ill effects.

The object is attained by providing an amino acid composition for suppressing the outbreak of functional disorders of a liver when alcohols are excessively taken, including threonine, proline, glycine, valine, isoleucine, leucine, tyrosine, phenylalanine, lysine, aspartic acid, serine, glutamic acid, alanine, methionine, tryptophane, histidine and arginine, and each amino acid is desirably contained in their respective mixing ratio, that is, 2 to 15 moles of the threonine, 4 to 30 moles of the proline, 7 to 20 moles of the glycine, 4 to 8 moles of the valine, 3 to 9 moles of the isoleucine, 2 to 12 moles of the leucine, 1 to 9 moles of the tyrosine, 0.5 to 5 moles of the phenylalanine, 5 to 11 moles of the lysine, 0.1 to 5 moles of the aspartic acid, 0.1 to 5 moles of the serine, 0.1 to 4 moles of the glutamic acid, 0.1 to 12 moles of the alanine, 0.1 to 5 moles of the methionine, 0.1 to 5 moles of the tryptophane, 0.1 to 5 moles of the histidine and 0.1 to 5 moles of the arginine. Another amino acid composition excluding the tryptophane from the above composition also has a function of suppressing the outbreak of the functional disorders of the liver when alcohols are excessively taken. The present invention also includes an amino acid composition solution, preferably, aqueous solution having substantially the same formulation as the above amino acid composition in a liquid phase.

As described earlier, it is clarified that the VAAM has the above-described action for accelerating kinesthetic functions by means of a complex action mechanism, and accelerates lipid metabolism. The present inventors have examined in detail whether or not the action for accelerating kinesthetic functions possessed by VAAM efficiently acts on the improvement of other diseases not directly connected with the action for accelerating kinesthetic functions

Wastes are likely to be accumulated in the liver having the above functions when exercise is extremely performed or alcohols are excessively taken, and when the functions are not properly accelerated, the outbreak of functional disorders of the liver may appear. Even after the outbreak thereof, the functional disorders may worsen and become unhealable in absence of a proper treatment.

The present inventors have found that the outbreak of functional disorders of the liver when alcohols are excessively taken can be suppressed by means of administration of VAAM having the specified formulation (different from that of the known VAAM) to an ordinary person before the outbreak of functional disorders of the liver.

The first amino acid composition in accordance with the present invention (hereinafter referred to as "first invention") contains 17 amino acids as essential components, and each amino acid is desirably contained in their respective mixing ratio, that is, 2 to 15 moles of the threonine, 4 to 30 moles of the proline, 7 to 20 moles of the glycine, 4 to 8 moles of the valine, 3 to 9 moles of the isoleucine, 2 to 12 moles of the leucine, 1 to 9 moles of the tyrosine, 0.5 to 5 moles of the phenylalanine, 5 to 11 moles of the lysine, 0.1 to 5 moles of the aspartic acid, 0.1 to 5 moles of the serine, 0.1 to 4 moles of the glutamic acid, 0.1 to 12 moles of the alanine, 0.1 to 5 moles of the methionine, 0.1 to 5 moles of the tryptophane, 0.1 to 5 moles of the histidine and 0.1 to 5 moles of the arginine. In addition to these amino acids, another amino acid, a water-soluble vitamin, an acid such as citric acid or a small amount of an additive may be contained.

The second amino acid composition in accordance with the present invention (hereinafter referred to as "second invention") contains 16 amino acids as essential components excluding the tryptophane from the amino acid composition of the first invention. The action of the tryptophane-free amino acid composition of the second invention is similar to or somewhat lesser than that of the first invention. Each amino acid is desirably an L-amino acid.

The amino acid composition or its solution in accordance with the first invention of the present invention has lower values of serum GOT and GPT, a lower content of liver triglyceride and a lower amount of total liver cholesterol than those of other amino acid compositions. The GOT, the GPT, the triglyceride and the total cholesterol are indexes for the actions for suppressing the outbreak of the functional disorders of the liver when alcohols are excessively taken. The above amino acid composition or solution has a lower death rate due to acute poisoning and a lower knockdown rate. Accordingly, the use of the amino acid composition prevents the outbreak of the functional disorders of the liver when alcohols are excessively taken.

The removal of one or more amino acids from the composition of the first invention hardly maintains the action for suppressing the outbreak of the functional disorders of the liver when alcohols are excessively taken. However, the removal of the tryptophane is an exception. The tryptophane-free amino acid composition or its solution in accordance with the second invention has the action for suppressing the outbreak of the functional disorders of the liver and may be satisfactorily used though the degree of the suppression is not as high as that of the first invention.

While the amino acid compositions of the present invention are effective for suppressing the outbreak of the functional disorders of the liver when alcohols are excessively taken as a whole, the compositions are especially effective for alcoholic functional disorders of a liver.

The amino acid compositions of the first and the second inventions may be taken in the powder form or in liquid form after being dissolved into water. The method for taking includes a general administration method such as oral administration, rectum administration, intravenous injection and intravenous feeding.

In case of the oral administration, the composition may be used as a powder, a granular medicine, a pill, a capsule and a lozenge after the composition is mixed with a support, a shaping agent and a diluting agent medically permissible in addition to the administration of the composition itself. However, the oral administration of the composition itself is preferable because a longer period of time is required for absorbing solid powder or a pill. In this case, the composition may be administrated in the solution with a proper additive such as a salt including sodium chloride, a pH adjuster and a chelating agent. In case of use in an injection, the composition may be dissolved in sterilized and distilled water after the addition of a proper buffer or an isotonic agent.

The time for taking is not especially restricted, and the composition may be taken in any time before or after alcohols are excessively taken. The composition is preferably taken before alcohols are excessively taken in the form of a solution such as health drinks (for example, soft drinks, powder drinks and medical drinks for purposes of nutritional fortification and supplementation).

Because of the lower toxicity, the administration amount of amino acid composition of the present invention can be established within a larger range. Depending on the method and the purpose of the usage, the composition may be usually administrated in 0. 5 to 5 g per dosage, preferably 1 to 2 g per dosage, and 1 to 20 g per day, preferably 4 to 10 g per day. In case of a solution, 10 to 1000 ml of solution containing 0.5 to 10 % by weight of the composition per dosage, preferably 100 to 400 ml of solution containing 1 to 4 % by weight of the composition per dosage is administrated or taken.

As apparent from the following Examples, the amino acid compositions of the first and the second inventions exhibit prominent suppressing actions against the outbreak of functional disorders of the liver when alcohols are excessivly taken. Further, the nature-originated amino acid composition which contains the 17 amino acids out of the 20 amino acids constituting a protein is lowly toxic and has extremely efficient action for suppressing the outbreak of functional disorders of the liver, when alcohols are excessively taken.

As described earlier, the amino acid composition of the present invention may be used in solution, especially in aqueous solution. In this case, the solution can be prepared by dissolving the composition of the first or the second invention as it is or by separately dissolving the individual amino acids into water to realize the amino acid composition in the solution.
Fig.1A is a graph showing a change in time of GOT concentration value when alcohol was administrated to a group of mice, and Fig.1B is a graph showing a change in time of GPT concentration value under the substantially same conditions.
Fig.2 is a graph showing GOT concentration values when each of amino acid compositions were administrated to a group of mice in which alcoholic functional disorders of a liver were developed
Fig.3 is a graph showing GPT concentration values when each of amino acid compositions were administrated to a group of mice in which alcoholic functional disorders of a liver were developed.
Fig.4 is a graph showing triglyceride amounts in a liver when each of amino acid compositions were administrated to a group of mice in which alcoholic functional disorders of the liver were developed.
Fig.5 is a graph showing total cholesterol amounts in a liver when each of amino acid compositions were administrated to a group of mice in which alcoholic functional disorders of the liver were developed.
Fig.6 is a graph showing a death rate due to acute poisoning when each of amino acid compositions were administrated to a group of mice in which alcoholic functional disorders of a liver were developed.
Figs. 7 is a graph showing a knockdown rate when each of amino acid compositions were administrated to a group of mice in which alcoholic functional disorders of a liver were developed.

Examples and Comparative Examples of tests for suppressing the outbreak of functional disorders of a liver when alcohols are excessivly taken by amino acid compositions of the present invention will be described. However, these Examples do not restrict the present invention.

Before describing the respective Examples in connection with the amino acid compositions, the concentration measurements for GOT, GPT, total cholesterol and triglyceride employed in Examples and Comparative Examples will be described.

### Measurements of GOT. GPT. Total Cholesterol and Triglyceride

In the present Examples, outbreak and healing of functional disorders of a liver were judged by employing GOT (Glutamic Oxaloacetic Transaminase), GPT (Glutamic Pyruvic Transaminase), total cholesterol and triglyceride which are indexes for functional disorders of the liver. GOT and GPT are contained in transaminase which is an enzyme catalyzing an amino group rearrangement between an amino acid and an a -keto acid. Among the indexes, the GOT and the GPT are clinically important, but in case of T -GPT, activity is an indetectable low value in solvents. The GOT is an enzyme catalyzing an amino group rearrangement between aspartic acid, α-keto glutaric acid and glutamic acid, oxalacetic acid. The GOT exists most frequently in myocardium, livers and brain and then in skeletal muscles and kidneys. The GPT is an enzyme catalyzing an amino group rearrangement between alanine, α-keto glutaric acid and glutamic acid, pyruvic acid. The GPT exists in livers and kidneys. Myocardial infarction and fulminated hepatitis significantly increase serum GOT. Acute hepatitis and chronic hepatitis significantly increase GPT. Accordingly, GOT and GPT are useful diagnostic indexes for these diseases.

The concentration of cholesterol (neutral fat) is intimately correlated with formation, absorption and catabolism of cholesterol in the liver and the intestinal tract, as well as metabolism of lipoprotein in blood.

Lipids in serum include triglyceride, cholesterol, phospholipid, liberated fatty acids and a small amount of lipophilic substances such as vitamins and carotin. The measurement of triglyceride in serum is an important inspection for the diagnosis of arteriosclerosis, coronary sclerosis and diabetes.

Measurements were conducted using a clinical diagnosis kit available from Wako Pure Chemical Industries, Ltd.

### [A] Measurement of GOT

The following reagents were used for measurement of GOT.

### Enzyme for GOT

An enzyme having the following formulation was dissolved and used.

| | |
|---|---|
| Pyruvic acid oxidase (POP) (originated from Pediococcus genus) | 5.4 unit/ml |
| Oxaloacetic acid decarboxylase (OAC) (originated from Pseudomonas genus) | 14 unit/ml |
| 4-aminoantipyrine | 2.0 mmol/liter |
| Ascorbic acid oxidase (originated from pumpkin) | 0.90 unit/ml |
| Catalase (originated from cow's liver) | 270 unit/ml |

### Substrate buffer for GOT

| | |
|---|---|
| 20mMGood (HEPES) buffer | pH7.0 |
| L- aspartic acid | 0.40 mol/liter |

### Color producing agent

During dissolution, 5.9 unit/ml of peroxidase (originated from horseradish) was used.

### Dissolution liquid for color producing agent

| | |
|---|---|
| 20mMGood (HEPES) buffer | pH7.0 |
| α-keto glutaric acid | 36mmol/liter |
| N-ethyl-N-(2-hydroxy-3-sulphopropyl)-m-toluidine sodium(TOOS) | 2.2mmol/liter |

### Reaction stopping liquid

| | |
|---|---|
| Citric acid | 50mmol/liter |
| Surface active agent | |

### Standard liquid for GOT

| | |
|---|---|
| Potassium pyruvate | |
| GOT | corresponding to 100 Karmen units |

Then, the principle of measuring GOT using these reagents will be described.

The action of the substrate enzyme liquid to a specimen to be measured generates glutamic acid and oxalacetic acid from L- aspartic acid and a -keto glutaric acid, respectively, by means of GOT in the specimen. The oxalacetic acid thus generated is converted into pyruvic acid by the action of the oxaloacetic acid decarboxylase (OAC). The pyruvic acid thus generated produces hydrogen peroxide by oxidation of the pyruvic acid by the action of the pyruvic acid oxidase (POP) in the presence of thiamine-pyrophosphoric acid (TPP) and flavin-adenine-dinucleotide (FAD). The hydrogen peroxide thus produced quantitatively and oxidatively condenses the TOOS and 4-aminoantipyrine to generate violet pigment (refer to Formula 1) by the action of the co-existing peroxidase (POD). Refer to Formula 1.

The active value of the GOT in the specimen is measured by the violet absorbance. The ascorbic acid contained in the specimen is decomposed by the ascorbic acid oxidase contained in the substrate enzyme liquid, thereby affecting little influence on the measured value. The pyruvic acid already existing in the serum is decomposed during preliminary heating at 37.5 °C.

### Measurement of GOT

GOT was measured in accordance with the following procedure.

After 0.25 ml of the substrate buffer was added to 0.01 ml of the specimen and heated for five minutes at 37 °C, 0.25 ml of the color producing agent was added thereto and heated at 37 °C for precisely 20 minutes. Finally, 0.1 ml of the reaction stopping agent was added and sufficiently agitated. Within 60 minutes, the absorbance of the specimen at 555 nm was measured by means of a spectrophotometer using a blind specimen (distilled water was added in place of the specimen) as reference.

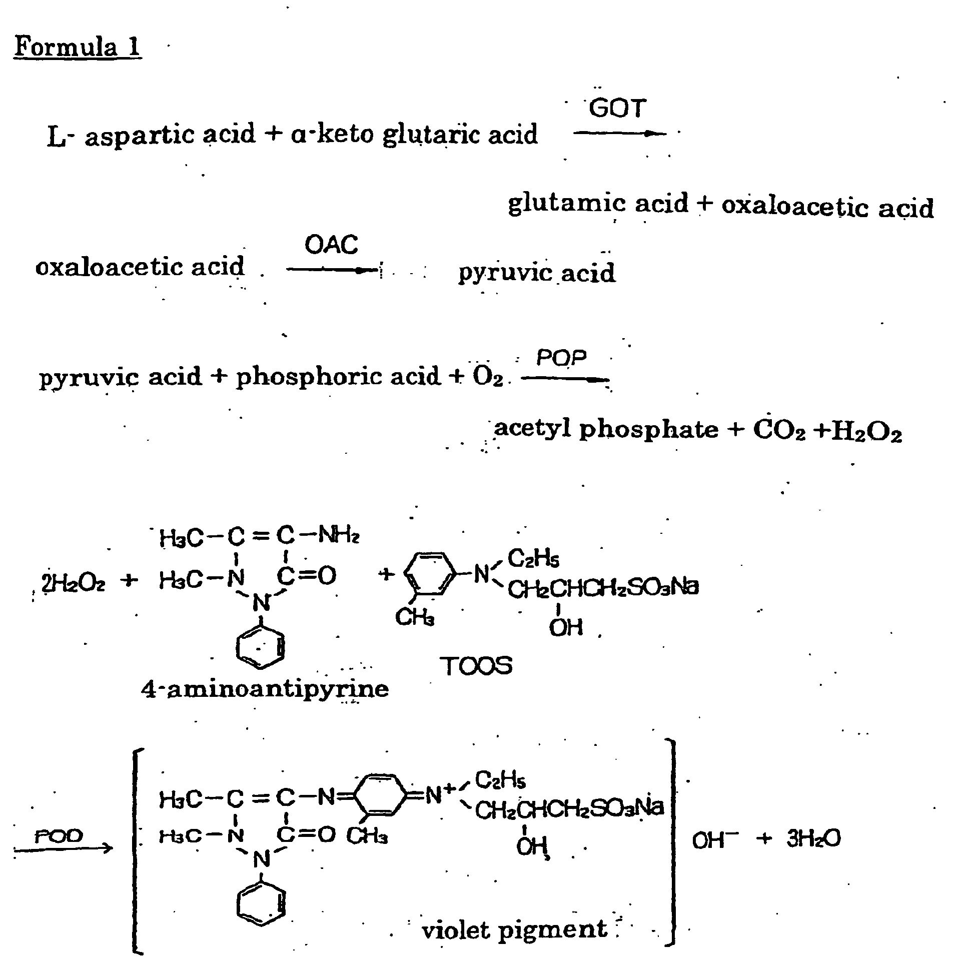

After a standard specimen having a known concentration prepared by using the standard liquid was preliminarily heated and the substrate buffer was added thereto, the absorbance of the standard specimen was measured and a calibration curve was prepared using the measured values.

### [B] Measurement of GPT

The following reagents were used for the measurement of GPT.

### Enzyme for GPT

An enzyme for the GPT having the following formulation was dissolved and used.

| | |
|---|---|
| Pyruvic acid oxidase (POP) (originated from Pediococcus genus) | 5.4 unit/ml |
| 4-aminoantipyrine | 2.0 mmol/liter |
| Ascorbic acid oxidase (originated from pumpkin) | 0.90 unit/ml |
| Catalase (originated from cow's liver) | 270 unit/ml |

### Substrate buffer for GPT

| | |
|---|---|
| 20mMGood (HEPES) buffer | pH7.0 |
| L- aspartic acid | 1.0 mol/liter |

### Color producing agent

During dissolution, 5.9 unit/ml of peroxidase (originated from horseradish) was used.

### Dissolution liquid for color producing agent

| | |
|---|---|
| 20mMGood (HEPES) buffer | pH7.0 |
| α -keto glutamic acid | 36mmol/liter |
| N-ethyl-N-(2-hydroxy-3-sulphopropyl)-m-toluidine sodium(TOOS) | 2.2mmol/liter |

### Reaction stopping liquid

| | |
|---|---|
| Citric acid | 50mmol/liter |
| Surface active agent | |

### Standard liquid for GPT

| | |
|---|---|
| potassium pyruvate | |
| GPT | corresponding to 100 Karmen units |

Then, the principle of measuring GPT using these reagents will be described.

The action of the substrate enzyme liquid to a specimen to be measured generates glutamic acid and pyruvic acid from L-aspartic acid and α-keto glutaric acid, respectively, by means of GPT in the specimen. The pyruvic acid thus generated produces hydrogen peroxide similarly to the above principle of measuring GOT, and finally produces violet pigment (refer to Formula 2).

The active value of GPT in the specimen is measured by the violet absorbance. Similarly to the GOT measurement, the ascorbic acid contained in the specimen is decomposed by the ascorbic acid oxidase contained in the substrate enzyme liquid, thereby affecting little influence on the measured value. The pyruvic acid already existing in the serum is decomposed during preliminary heating at 37.5 °C.

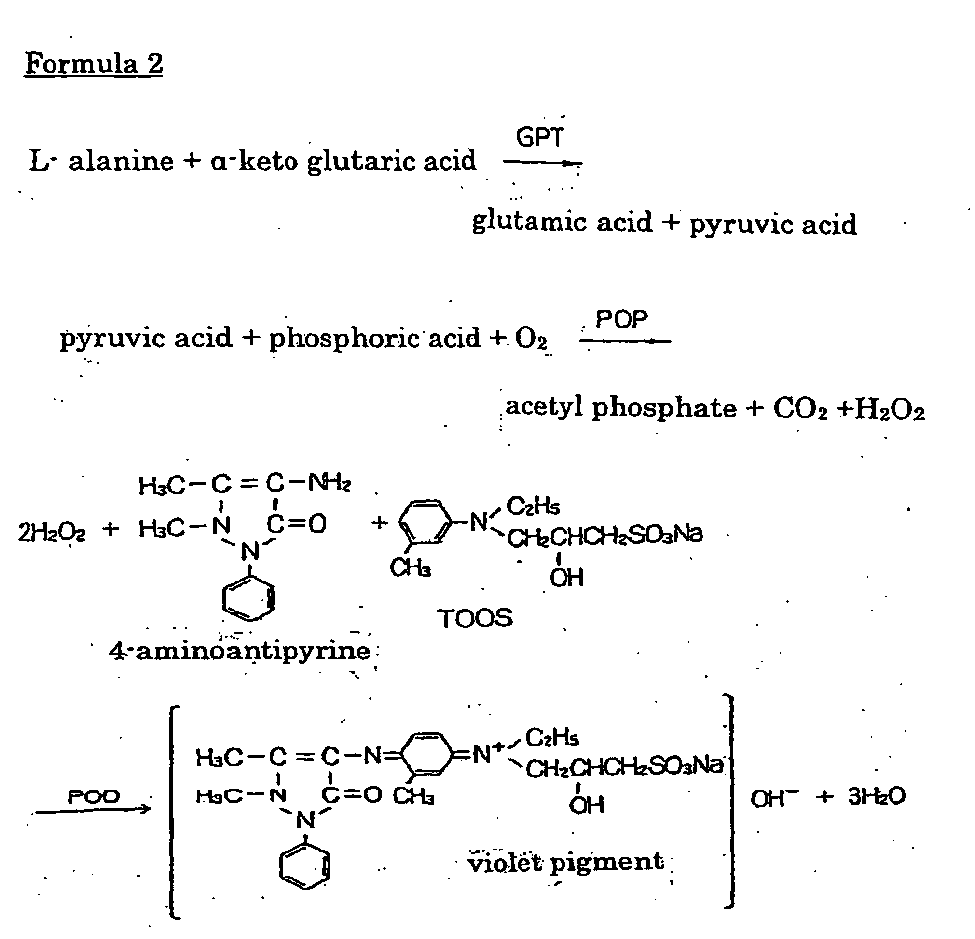

### Measurement of GPT

The measurement of GPT was conducted similarly to that of the GOT by using a calibration curve separately prepared.

### [C] Measurement of total cholesterol

The following reagents were used for the measurement of the total cholesterol.

### Buffer solution

| | |
|---|---|
| 0.1 M phosphoric acid buffer solution | pH 7.0 |
| Phenol | 10.6 mmol/liter |

### Color producing agent

| | |
|---|---|
| During dissolution, the following reagents were used. | |
| Cholesterol esterase (originated from Candida genus) | 0.12 unit/ml |
| Cholesterol oxidase (originated from Streptomyces genus) | 0.12 unit/ml |
| Peroxidase (originated from horseradish) | 4.3 unit/ml |
| 4-aminoantipyrine | 0.74 mmol/liter |

### Standard serum

Serum (originated from cow)

Then, the principle of measuring total cholesterol using these reagents will be described.

The action of the color generating agent to a specimen to be measured decomposes the cholesterols into the liberated cholesterols and fatty acids by the action of the cholesterol esterase. The cholesterols thus generated together with the liberated cholesterols already existing are oxidized to produce hydrogen peroxide by means of the action of the cholesterol oxidase. The hydrogen peroxide thus produced quantitatively and oxidatively condenses the phenols and 4-aminoantipyrine to generate red pigment (red quinone-based pigment, refer to Formula 3) by the action of the peroxidase (POD). Refer to Formula 3.

The concentration of the total cholesterol in the specimen was determined by measuring the red absorbance.

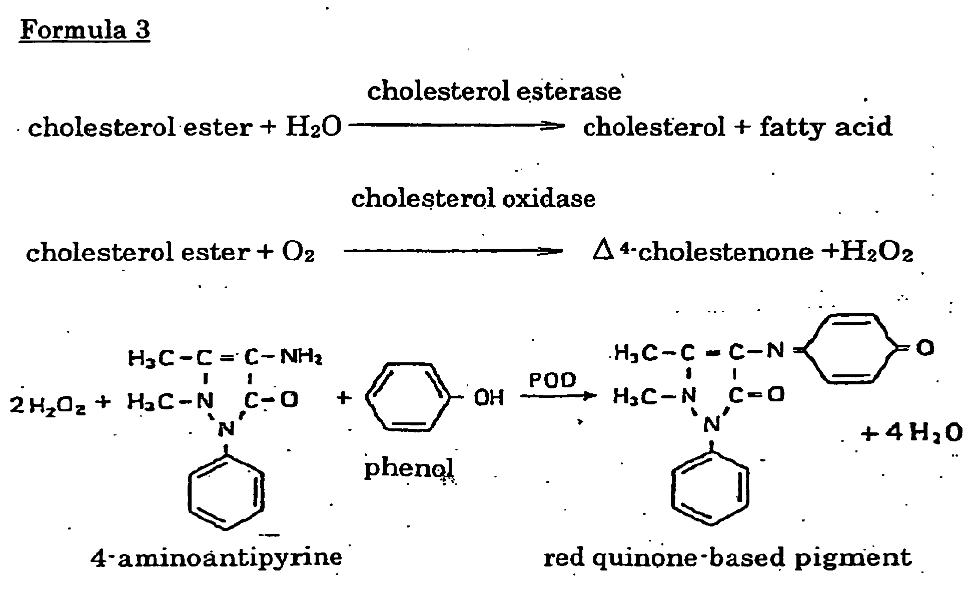

### Measurement of total cholesterol

After 0.01 ml of the specimen was dissolved in isopropanol, 1.5ml of the color producing agent was added therein followed by heating at 37 °C for 10 minutes under sufficient agitation. The absorbance of the specimen at 505 nm was measured by using a blind specimen as reference by means of a spectrophotometer.

After a standard specimen having a known concentration prepared by using the standard liquid was preliminarily heated and the substrate buffer was added thereto, the absorbance of the standard specimen was measured and a calibration curve was prepared using the measured values.

### [D] Measurement of triglyceride

The following reagents were used for the measurement of the triglyceride.

### Buffer solution

| | |
|---|---|
| 50 mM tris-buffer solution | pH 7.5 |
| P-chlorophenol | 5.4 mmol/liter |

### Color producing agent

During dissolution, the following reagents were used.

| | |
|---|---|
| Lipoprotein lipase (originated from Chromobacterium genus) | 36 unit/ml |
| Adenosine-5'-triphosphoric acid bisodium trihydrate (ATP) (Bacterium) | 1.8 mmol/liter |
| Glycerol kinase (originated from Streptomyces genus) | 2.2 unit/ml |
| Glycerol-3-phosphoric acid oxidase (GPO) (originated from Aerococus genus) | 4.4 unit/ml |
| Peroxidase (originated from horseradish) | 2.1 unit/ml |
| 4-aminoantipyrine | 0.69 mmol/liter |

### Standard liquid

| | |
|---|---|
| Glycerin | 31.2mg/dl |
| Triolein | corresponding to 300mg/dl |

Then, the principle of measuring the triglyceride by using these reagents will be described.

The action of the lipoprotein lipase (LPL) decomposes the triglyceride in the specimen to be measured into glycerin and a fatty acid. The glycerin thus generated is converted into glycerol-3-phosphoric acid by the action of the glycerol kinase (GK) in the presence of ATP. The glycerol-3-phosphoric acid is oxidized by the action of glycerol-3-phosphoric acid oxidase (GPO), thereby producing hydrogen peroxide. The hydrogen peroxide thus produced quantitatively and oxidatively condenses the p-chlorophenol and the 4-aminoantipyrine to generate red pigment (red quinone-based pigment, refer to Formula 4) by the action of the peroxidase (POD). Refer to Formula 4.

The concentration of the triglyceride in the specimen was determined by measuring the red absorbance.

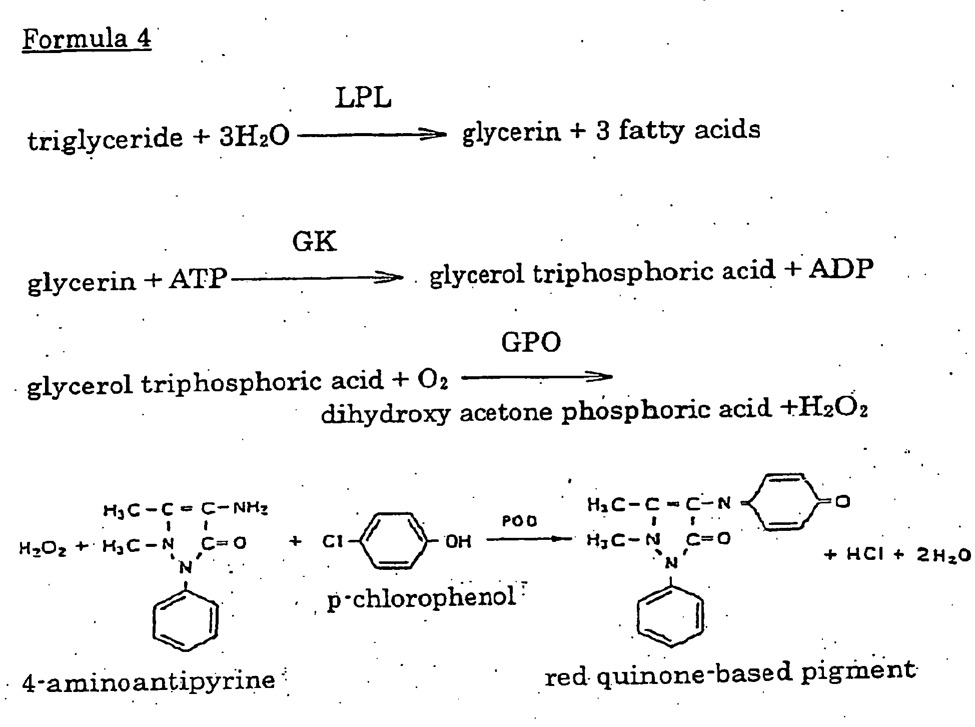

### Measurement of triglyceride

The measurement of the triglyceride was conducted similarly to that of the total cholesterol.

Then, the effect for suppressing the outbreak of the functional disorders of the liver when alcohols are excessivly taken generated by each of the above amino acid compositions was confirmed by using the above measuring methods.

### Example 1

Male mice (ddY, SPF), six to eight weeks from birth were divided into several groups each having five mice and were bred in a clean room by supplying an ordinary feed without limit. The same nutrient solution was administered to the respective 10 to 12 mice in Examples 1 and 2 and Comparative Examples 1 to 10.

The nutrient solution containing a formulation (VAAM) having 17 amino acids in a specified ratio shown in Example 1 of Table 1 was prepared by mixing commercially available amino acids and dissolving in water. The nutrient solution was orally administered to the above mice every 12 hours such that 37.5 µl of the solution was fed per 1 g of the body weight of mice. The same conditions were maintained from first to fifth administrations. At the sixth administration, the same amount of 25% ethanol was administrated in place of the nutrient solution, thereby compulsively generating alcoholic functional disorders of the liver. The administration was stopped, and after 12 hours from the ethanol administration, blood was drawn from abdominal vena cava. After the blood was allowed to stand at room temperature for 30 minutes after the drawing, the serum was centrifugally separated. The concentrations of GOT and GPT were measured in accordance with the above described methods.

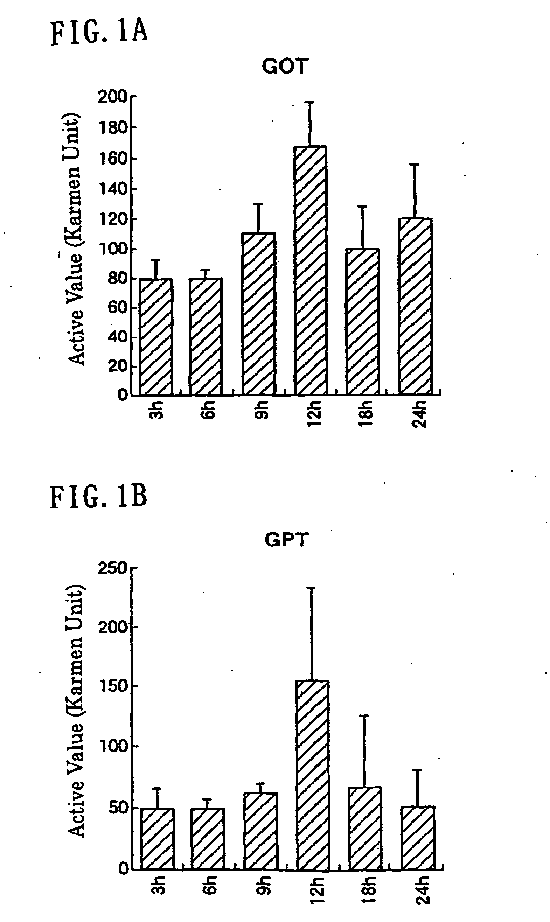

The change in time of the GOT concentration when only the 25% ethanol was administrated is shown in Fig.1A and that of the GPT concentration is shown in Fig.1B.

As apparent from Figs.1A and 1B, the GOT and the GPT concentrations reached maximum values, that is, about 165 Karmen units and about 160 Karmen units, respectively, after 12 hours following the ethanol administration, and then decreased. The "1 Karmen unit" is defined such that decrease of absorbance at 340 nm at 25 °C per minute by means of 1ml of the serum is 0.001.

In the VAAM administered groups, the amounts of triglyceride and the total cholesterol in the liver after 12 hours following the ethanol administration were measured to be about 390 mg and about 80 mg (average values), respectively.

Further, the death rate due to acute poisoning and the knockdown rate of the mice after 12 hours following the ethanol administration were measured to be about 6 % and about 12 %, respectively.

**Table 1**

| **Amino Acid** (molar%) | **Examples** | | **Comparative Examples** | | | | |
|---|---|---|---|---|---|---|---|
| | 1 | 2 | 1 | 2 | 3 | 4 | 5 |
| | VAAM | V-Trp | CAAM | V-1 | V-9 | V-Tyr | V-Lys |
| Asp | 0.2 | 0.2045 | 7.5 | - | - | 0.213 | 0.2045 |
| Thr | 7.2 | 7.3620 | 2.5 | 8.29 | - | 7.66 | 7.8775 |
| Ser | 2.5 | 2.5562 | 8.0 | - | - | 2.66 | 2.7352 |
| Glu | 3.2 | 3.2720 | 19.6 | - | - | 3.404 | 3.5011 |
| Pro | 18.0 | 18.405 | 8.5 | 20.44 | 41.2 | 19.15 | 19.694 |
| Gly | 19.1 | 19.530 | 4.5 | 19.83 | - | 20.32 | 20.897 |
| Ala | 6.0 | 6.1350 | 4.5 | 5.81 | 13.2 | 6.383 | 6.5646 |
| Val | 5.9 | 6.0327 | 5.5 | 5.44 | 12.6 | 6.277 | 6.4551 |
| Cys | **-** | - | 0.4 | - | - | - | - |
| Met | 0.5 | 0.5112 | 2.5 | - | - | 0.532 | 0.5470 |
| Ile | 4.5 | 4.6012 | 5.5 | 5.67 | 9.8 | 4.787 | 4.9234 |
| Leu | 6.2 | 6.3395 | 8.5 | 5.96 | - | 6.596 | 6.7834 |
| Tyr | 6.0 | 6.1350 | 5.0 | 5.78 | **-** | - | 6.5646 |
| Phe | 3.8 | 3.8855 | 4.0 | 3.62 | - | 4.043 | 4.1575 |
| Lys | 8.6 | 8.7935 | 7.0 | 8.37 | 23.2 | 9.149 | - |
| Trp | 2.2 | - | 1.0 | 1.92 | - | 2.34 | 2.4070 |
| His | 2.6 | 2.6585 | 2.5 | 3.05 | - | 2.766 | 2.8446 |
| Arg | 3.5 | 3.5787 | 3.0 | 3.47 | - | 3.723 | 3.8293 |

### Example 2 and Comparative Examples 1 to 10

The results in Example 1 revealed that GOT and GPT concentrations reached maximum 12 hours following the ethanol administration.

The amino acid compositions having the formulation specified in Table 1, that is, VAAM (Example 1), V-Trp (Example 2), CAAM (Comparative Example 1), V-1 (Comparative Example 2), V-9 (Comparative Example 3), V-Try (Comparative Example 4) and V-Lys (Comparative Example 5) were administrated to the mice under the same conditions as those of Example 1, and then the concentrations of GOT, GPT, triglyceride and total cholesterol were measured 12 hours following the ethanol administration.

Although not shown in Table 1, the same measurements were conducted to D.W. (Comparative Example 6, distilled water), 2% Trp (Comparative Example 7, 2% tryptophane aqueous solution), 0.25% Tyr (Comparative Example 8, 0.25% tyrosine aqueous solution), 2% Lys (Comparative Example 9, 2% lysine aqueous solution) and control (Comparative Example 10, the concentration was measured after the blood of the mice bred under the same conditions was drawn except that the administration of the amino acids, the nutrition solution, the water and the alcohol was not conducted).

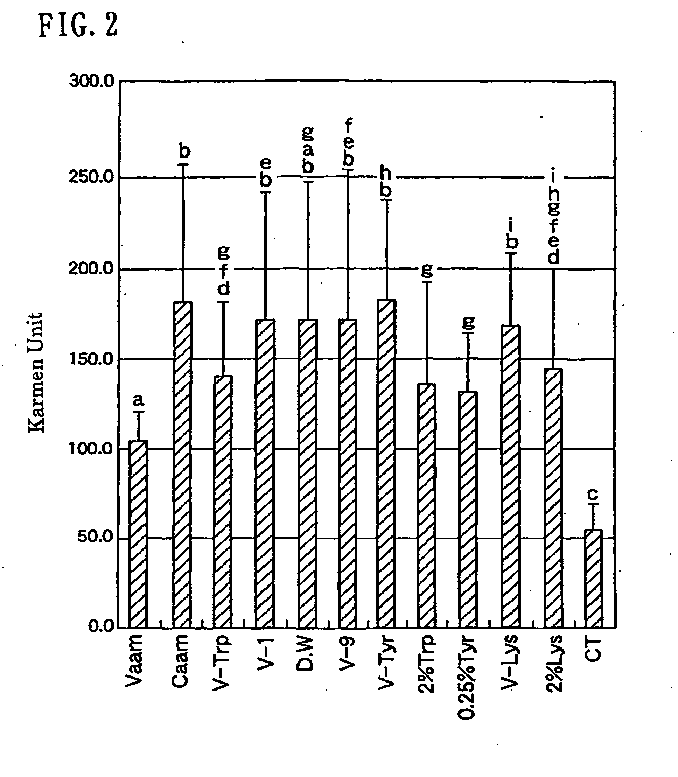

The GOT concentrations, the GOT concentrations, the triglyceride concentrations and the total cholesterol concentrations of each amino acid composition are shown in Fig.2, Fig.3, Fig.4 and Fig.5, respectively.

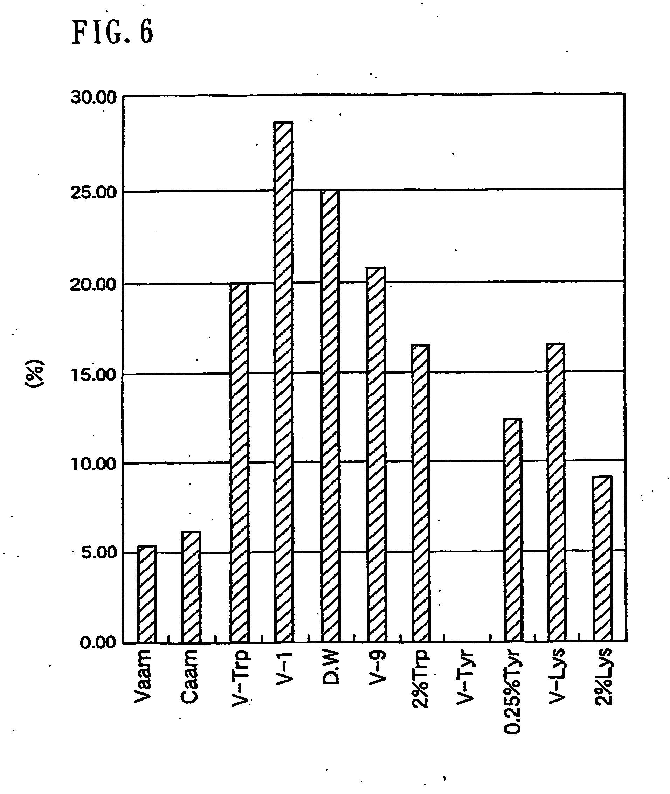

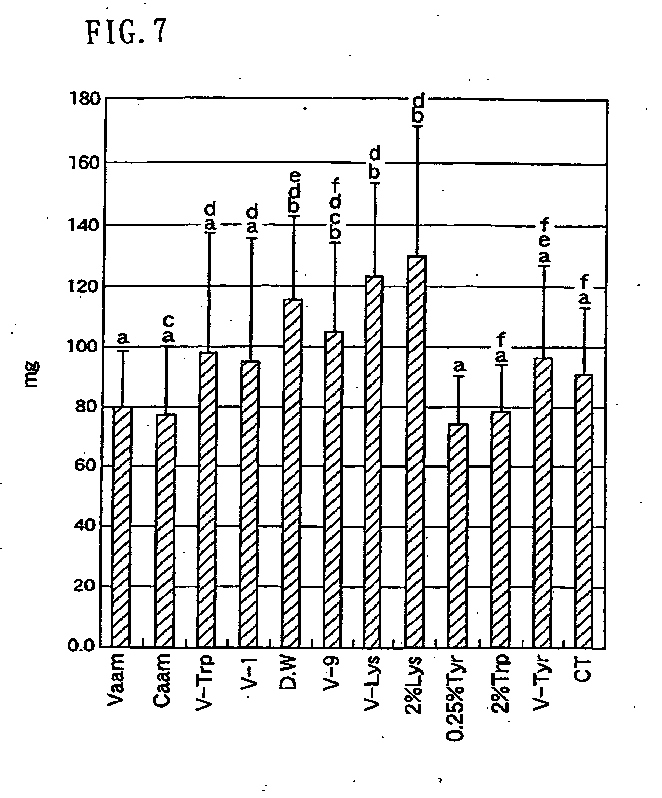

Further, the death rate due to acute poisoning and the knockdown rate of the mice to which each of amino acid compositions was administrated are shown in Fig.6 and Fig.7, respectively.

### Consideration on Examples 1 and 2 and Comparative Examples 1 to 10

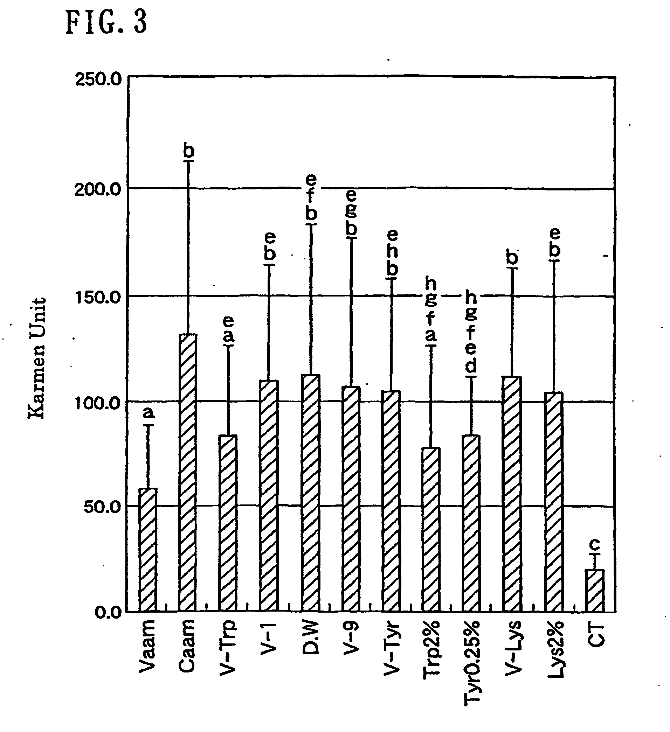

Among the amino acid compositions of Examples and Comparative Examples, the VAAM group of Example 1 had the smallest increases of GOT (Fig.2) and GPT (Fig.3) and the values thereof were significantly lower than those of the other amino acid compositions.

The V-Trp group of Example 2 had GOT values significantly higher than those of VAAM. However, the V-Trp group had GOT values nearer to those of the 2% Trp, 0.25%Tyr and 2% Lys groups, and significantly lower than those of amino acid compositions other than those above. In connection with the GPT value (Fig.3), the V-Trp had GPT value nearer to those of 2% Trp and 0.25%Tyr and significantly lower than those of the amino acid compositions other than those above.

From the above results, it could be judged that the action of VAAM for suppressing the outbreak of functional disorders of the liver when alcohols are excessivly taken using GOT and GPT as the indexes, was superior. Following VAAM the next most effective was V-Trp, 2% Trp and then 0.25% Tyr.

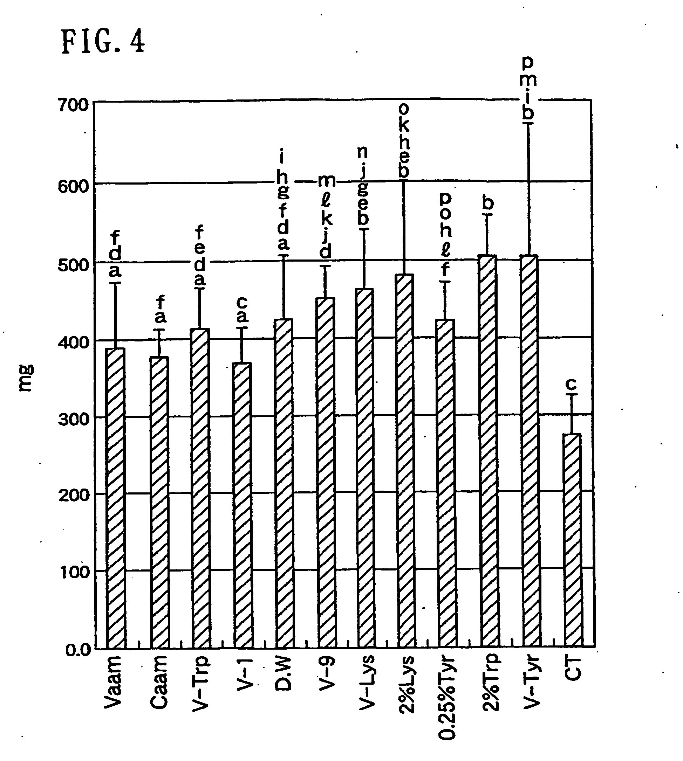

In connection with the triglycerides (amounts of neutral fat) shown in Fig.4, each of the amino acid compositions exhibited relatively higher values as a whole, and no significant difference was observed among the values for VAAM, CAAM, V-Trp and D.W. While the amounts of triglycerides thereof were smaller than those of the other amino acid compositions, no significant difference such as in the case for GOT and GPT was observed. However, it could be conjectured that the fat is burnt especially in the VAAM group, thereby decreasing the accumulation of fat to reduce fatty livers.

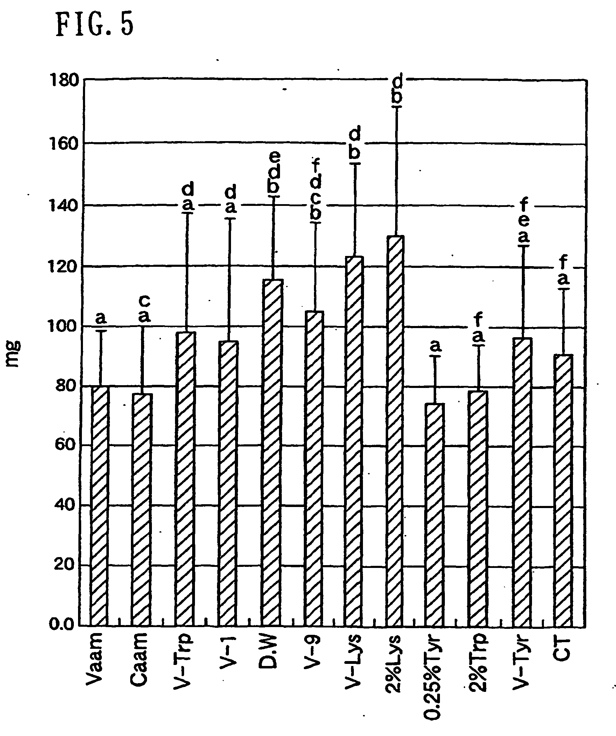

In connection with the total cholesterol shown in Fig.5, the amount in the alcohol administrated group did not reach significantly higher values than in the control (CT). However, VAAM, CAAM, 0.25% Tyr and 2% Trp exhibited values lower than those of the control, and the other amino acid compositions, including the D.W., exhibited significantly higher values.

In connection with the death rate due to acute poisoning shown in Fig.6, the death rate of the V-Tyr group was zero and the death rates of the VAAM group and the CAAM group were slightly higher than 5%. The death rates of the 2% Trp group and the V-Lys group exceeded 15%, that of the V-Trp group reached 20%, and that of the V-9 group exceeded 20%. Further, the death rate of the D.W. group reached 25%, and that of the V 1 group reached around 30%. As described, the toxicity for VAAM administered mice was smaller than those for the other amino acid compositions.

In connection with the knockdown rate shown in Fig.7, the rate of the VAAM administered group was lowest, and the rates of the other amino acid compositions reached values 2.5 to 7 times that of the VAAM group. Especially, the V-Tyr group for which the death rate was zero had a knockdown rate near 70% which was the highest among the amino acid compositions. These results showed that VAAM most excellently suppressed acute alcoholic poisoning.

The above results comprehensively and apparently show that VAAM suppresses alcoholic functional disorders of the liver more remarkably than the other amino acid compositions and function recovery of the whole mouse also resulted. V-Trp prepared by excluding tryptophane from VAAM has the action for suppressing the outbreak of functional disorders of the liver though it is not as much as that of VAAM. However, the further removal of any one of the amino acids from V-Trp weakened the improving action, thereby providing only insufficient improving action.

## Claims

1. An amino acid composition or solution for use in suppressing the outbreak of functional disorders of the liver when alcohols are excessively taken,
wherein the amino acid composition or solution comprises threonine, proline, glycine, valine, isoleucine, leucine, tyrosine, phenylalanine, lysine, aspartic acid, serine, glutamic acid, alanine, methionine, tryptophane, histidine and arginine, and wherein each of the amino acids is contained in such a molar ratio as 2 to 15 moles of threonine, 4 to 30 moles of proline, 7 to 20 moles of glycine, 4 to 8 moles of valine, 3 to 9 moles of isoleucine, 2 to 12 moles of leucine, 1 to 9 moles of tyrosine, 0.5 to 5 moles of phenylalanine, 5 to 11 moles of lysine, 0.1 to 5 moles of aspartic acid, 0.1 to 5 moles of serine, 0.1 to 4 moles of glutamic acid, 0.1 to 12 moles of alanine, 0.1 to 5 moles of methionine, 0.1 to 5 moles of tryptophane, 0.1 to 5 moles of histidine and 0.1 to 5 moles of arginine.

2. A tryptophane-free amino acid composition or solution for use in suppressing the outbreak of functional disorders of the liver when alcohols are excessively taken,
wherein said tryptophane-free amino acid composition or solution comprises threonine, proline, glycine, valine, isoleucine, leucine, tyrosine, phenylalanine, lysine, aspartic acid, serine, glutamic acid, alanine, methionine, histidine and arginine, and
wherein each of the amino acids is contained in such a molar ratio that 2 to 15 moles of threonine, 4 to 30 moles of proline, 7 to 20 moles of glycine, 4 to 8 moles of valine, 3 to 9 moles of isoleucine, 2 to 12 moles of leucine, 1 to 9 moles of tyrosine, 0.5 to 5 moles of phenylalanine, 5 to 11 moles of lysine, 0.1 to 5 moles of aspartic acid, 0.1 to 5 moles of serine, 0.1 to 4 moles of glutamic acid, 0.1 to 12 moles of alanine, 0.1 to 5 moles of methionine, 0.1 to 5 moles of-histidine and 0.1 to 5 moles of arginine.

3. The composition or solution of claim 1 or 2, wherein the functional disorders are alcoholic functional disorders.

4. The composition or solution of any one of claims 1 to 3, wherein the medicament further comprises a pharmaceutical excipient for oral administration of the medicament.

5. The composition or solution of any one of claims 1 to 4, wherein said amino acids are in a dose of 0.5 to 5g.

6. The composition or solution of any one of claims 1 to 5, wherein said medicament is in a form selected from the group consisting of powder, pill, capsule, granular, and lozenge.

7. The composition or solution of any of claims 1 to 5, wherein said medicament is in a form of 10 to 1000 ml solution containing 0.5 to 10% by weight of said amino acids.

8. The composition or solution of any one of claims 1 to 7, wherein said amino acids are in a daily dosage form of 1 to 20g per day.

9. Use of an amino acid composition or solution as defined in any one of claim 1 to 8 for the manufacture of a medicament for suppressing the outbreak of functional disorders of the liver when alcohols are excessively taken.

## Patentansprüche

1. Aminosäurezusammensetzung oder -lösung zur Verwendung in der Unterdrückung des Ausbruchs von funktionellen Störungen der Leber, wenn Alkohol übermäßig eingenommen wird,
wobei die Aminosäurezusammensetzung oder -lösung Threonin, Prolin, Glycin, Valin, Isoleucin, Leucin, Tyrosin, Phenylalanin, Lysin, Asparaginsäure, Serin, Glutaminsäure, Alanin, Methionin, Tryptophan, Histidin und Arginin umfasst, und wobei jede der Aminosäuren in einem Molverhältnis von 2 bis 15 Mol Threonin, 4 bis 30 Mol Prolin, 7 bis 20 Mol Glycin, 4 bis 8 Mol Valin, 3 bis 9 Mol Isoleucin, 2 bis 12 Mol Leucin, 1 bis 9 Mol Tyrosin, 0,5 bis 5 Mol Phenylalanin, 5 bis 11 Mol Lysin, 0,1 bis 5 Mol Asparaginsäure, 0,1 bis 5 Mol Serin, 0,1 bis 4 Mol Glutaminsäure, 0,1 bis 12 Mol Alanin, 0,1 bis 5 Mol Methionin, 0,1 bis 5 Mol Tryptophan, 0,1 bis 5 Mol Histidin und 0,1 bis 5 Mol Arginin enthalten ist.

2. Tryptophan-freie Aminosäurezusammensetzung oder -lösung zur Verwendung in der Unterdrückung des Ausbruchs von funktionellen Störungen der Leber, wenn Alkohol übermäßig eingenommen wird,
wobei die Tryptophan-freie Aminosäurezusammensetzung oder -lösung Threonin, Prolin, Glycin, Valin, Isoleucin, Leucin, Tyrosin, Phenylalanin, Lysin, Asparaginsäure, Serin, Glutaminsäure, Alanin, Methionin, Histidin und Arginin umfasst, und
wobei jede der Aminosäuren in einem Molverhältnis von 2 bis 15 Mol Threonin, 4 bis 30 Mol Prolin, 7 bis 20 Mol Glycin, 4 bis 8 Mol Valin, 3 bis 9 Mol Isoleucin, 2 bis 12 Mol Leucin, 1 bis 9 Mol Tyrosin, 0,5 bis 5 Mol Phenylalanin, 5 bis 11 Mol Lysin, 0,1 bis 5 Mol Asparaginsäure, 0,1 bis 5 Mol Serin, 0,1 bis 4 Mol Glutaminsäure, 0,1 bis 12 Mol Alanin, 0,1 bis 5 Mol Methionin, 0,1 bis 5 Mol Histidin und 0,1 bis 5 Mol Arginin enthalten ist.

3. Zusammensetzung oder Lösung gemäß Anspruch 1 oder 2, wobei die funktionellen Störungen alkoholbedingte funktionelle Störungen sind.

4. Zusammensetzung oder Lösung gemäß einem der Ansprüche 1 bis 3, wobei das Medikament ferner einen pharmazeutischen Exzipienten zur oralen Verabreichung des Medikaments umfasst.

5. Zusammensetzung oder Lösung gemäß einem der Ansprüche 1 bis 4, wobei die Aminosäuren in einer Dosierung von 0,5 bis 5g vorliegen.

6. Zusammensetzung oder Lösung gemäß einem der Ansprüche 1 bis 5, wobei das Medikament in einer Form ist, die aus der Gruppe bestehend aus Pulver, Dragee (pill), Kapsel, Granulat und Pastille ausgewählt ist.

7. Zusammensetzung oder Lösung gemäß einem der Ansprüche 1 bis 5, wobei das Medikament in der Form einer Lösung von 10 bis 1000 ml vorliegt, welche 0,5 bis 10 Gewichtsprozent an Aminosäuren enthält.

8. Zusammensetzung oder Lösung gemäß einem der Ansprüche 1 bis 7, wobei die Aminosäuren in einer täglichen Dosierungsform von 1 bis 20g pro Tag vorliegen.

9. Verwendung einer Aminosäurezusammensetzung oder -lösung wie in einem der Ansprüche 1 bis 8 definiert zur Herstellung eines Medikaments zur Unterdrückung des Ausbruchs von funktionellen Störungen der Leber, wenn Alkohol übermäßig eingenommen wird.

## Revendications

1. Composition ou solution d'acides aminés destinée à être utilisée pour supprimer la survenue de troubles fonctionnels du foie en cas de consommation excessive d'alcools,
dans laquelle la composition ou solution d'acides aminés comprend de la thréonine, proline, glycine, valine, isoleucine, leucine, tyrosine, phénylalanine, lysine, acide aspartique, sérine, acide glutamique, alanine, méthionine, tryptophane, histidine et arginine, et où chacun des acides aminés est contenu selon un ratio molaire tel que 2 à 15 moles de thréonine, 4 à 30 moles de proline, 7 à 20 moles de glycine, 4 à 8 moles de valine, 3 à 9 moles d'isoleucine, 2 à 12 moles de leucine, 1 à 9 moles de tyrosine, 0,5 à 5 moles de phénylalanine, 5 à 11 moles de lysine, 0,1 à 5 moles d'acide aspartique, 0,1 à 5 moles de sérine, 0,1 à 4 moles d'acide glutamique, 0,1 à 12 moles d'alanine, 0,1 à 5 moles de méthionine, 0,1 à 5 moles de tryptophane, 0,1 à 5 moles d'histidine et 0,1 à 5 moles d'arginine.

2. Composition ou solution d'acides aminés exempte de tryptophane destinée à être utilisée pour supprimer la survenue de troubles fonctionnels du foie en cas de consommation excessive d'alcools,
dans laquelle ladite composition ou solution d'acides aminés exempte de tryptophane comprend de la thréonine, proline, glycine, valine, isoleucine, leucine, tyrosine, phénylalanine, lysine, acide aspartique, sérine, acide glutamique, alanine, méthionine, histidine et arginine, et
dans laquelle chacun des acides aminés est contenu selon un ratio molaire tel que 2 à 15 moles de thréonine, 4 à 30 moles de proline, 7 à 20 moles de glycine, 4 à 8 moles de valine, 3 à 9 moles d'isoleucine, 2 à 12 moles de leucine, 1 à 9 moles de tyrosine, 0,5 à 5 moles de phénylalanine, 5 à 11 moles de lysine, 0,1 à 5 moles d'acide aspartique, 0,1 à 5 moles de sérine, 0,1 à 4 moles d'acide glutamique, 0,1 à 12 moles d'alanine, 0,1 à 5 moles de méthionine, 0,1 à 5 moles d'histidine et 0,1 à 5 moles d'arginine.

3. Composition ou solution selon la revendication 1 ou 2, dans laquelle les troubles fonctionnels sont des troubles fonctionnels liés à l'alcool.

4. Composition ou solution selon l'une quelconque des revendications 1 à 3, dans laquelle le médicament comprend en outre un excipient pharmaceutique pour une administration orale du médicament.

5. Composition ou solution selon l'une quelconque des revendications 1 à 4, dans laquelle lesdits acides aminés sont en une dose de 0,5 à 5 g.

6. Composition ou solution selon l'une quelconque des revendications 1 à 5, dans laquelle ledit médicament est sous une forme sélectionnée dans le groupe consistant en une poudre, une pilule, une capsule, granulé et une pastille.

7. Composition ou solution selon l'une quelconque des revendications 1 à 5, dans laquelle ledit médicament est sous la forme d'une solution de 10 à 1000 ml contenant 0,5 à 10 % en poids desdits acides aminés.

8. Composition ou solution selon l'une quelconque des revendications 1 à 7, dans laquelle lesdits acides aminés sont sous une forme posologique quotidienne de 1 à 20 g par jour.

9. Utilisation d'une composition ou d'une solution d'acides aminés selon l'une quelconque des revendications 1 à 8, pour la fabrication d'un médicament destiné à supprimer la survenue de troubles fonctionnels du foie en cas de consommation excessive d'alcools.
